# EUROPEAN PATENT SPECIFICATION

(11) **EP 4 587 079 B1**
(45) Date of publication and mention of the grant of the patent: **12.08.2026**
(21) Application number: 23785908.7
(22) Date of filing: 12.09.2023
(51) Int. Cl.: A61M 1/28

(54) **DIALYSIS SYSTEM HAVING EXTERNAL TUBING SURFACE CLEANING**
DIALYSESYSTEM MIT REINIGUNG DER LEITUNGSAUSSENFLÄCHE
SYSTÈME DE DIALYSE AVEC NETTOYAGE DE LA SURFACE EXTÉRIEURE DES TUYAUX

(30) Priority: 14.09.2022 IN 202241052404
(43) Date of publication of application: 23.07.2025
(73) Proprietor: Vantive US Healthcare LLC, Deerfield, IL 60015 (US); Vantive Health GmbH, 8152 Glattpark (Opfikon) (CH)
(72) Inventor: APORVA, Raj Makam Ravindranath, Bangalore, Karnataka 560048 (IN); AKHILESH, Sham Sundar Vibhute, Bangalore, Karnataka 560048 (IN); SACHIN, Sunkad, Bangalore, Karnataka 560048 (IN)
(74) Representative: Potter Clarkson
(86) International application number: PCT/US2023/032477
(87) International publication number: WO 2024/059030

(56) References cited:
- CN-A- 111 760 105
- US-A1- 2007 161 946
- US-A1- 2022 203 005

## Description

### BACKGROUND

The present disclosure relates generally to medical fluid treatments and in particular to dialysis fluid treatments that require the pumping of patient-injectable fluids.

Due to various causes, a person's renal system can fail. Renal failure produces several physiological derangements. It is no longer possible to balance water and minerals or to excrete daily metabolic load. Toxic end products of metabolism, such as, urea, creatinine, uric acid and others, may accumulate in a patient's blood and tissue.

Reduced kidney function and, above all, kidney failure is treated with dialysis. Dialysis removes waste, toxins, and excess water from the body that normal functioning kidneys would otherwise remove. Dialysis treatment for replacement of kidney functions is critical to many people because the treatment is lifesaving.

One type of kidney failure therapy is Hemodialysis ("HD"), which in general uses diffusion to remove waste products from a patient's blood. A diffusive gradient occurs across the semi-permeable dialyzer between the blood and an electrolyte solution called dialysate or dialysis fluid to cause diffusion.

Hemofiltration ("HF") is an alternative renal replacement therapy that relies on a convective transport of toxins from the patient's blood. HF is accomplished by adding substitution or replacement fluid to the extracorporeal circuit during treatment. The substitution fluid and the fluid accumulated by the patient in between treatments is ultrafiltered over the course of the HF treatment, providing a convective transport mechanism that is particularly beneficial in removing middle and large molecules.

Hemodiafiltration ("HDF") is a treatment modality that combines convective and diffusive clearances. HDF uses dialysis fluid flowing through a dialyzer, similar to standard hemodialysis, to provide diffusive clearance. In addition, substitution solution is provided directly to the extracorporeal circuit, providing convective clearance.

Most HD, HF, and HDF treatments occur in centers. A trend towards home hemodialysis ("HHD") exists today in part because HHD can be performed daily, offering therapeutic benefits over in-center hemodialysis treatments, which occur typically bi- or triweekly. Studies have shown that more frequent treatments remove more toxins and waste products and render less interdialytic fluid overload than a patient receiving less frequent but perhaps longer treatments. A patient receiving more frequent treatments does not experience as much of a down cycle (swings in fluids and toxins) as does an in-center patient, who has built-up two or three days' worth of toxins prior to a treatment. In certain areas, the closest dialysis center can be many miles from the patient's home, causing door-to-door treatment time to consume a large portion of the day. Treatments in centers close to the patient's home may also consume a large portion of the patient's day. HHD can take place overnight or during the day while the patient relaxes, works or is otherwise productive.

Another type of kidney failure therapy is peritoneal dialysis ("PD"), which infuses a dialysis solution, also called dialysis fluid, into a patient's peritoneal chamber via a catheter. The dialysis fluid is in contact with the peritoneal membrane in the patient's peritoneal chamber. Waste, toxins, and excess water pass from the patient's bloodstream, through the capillaries in the peritoneal membrane, and into the dialysis fluid due to diffusion and osmosis, i.e., an osmotic gradient occurs across the membrane. An osmotic agent in the PD dialysis fluid provides the osmotic gradient. Used or spent dialysis fluid is drained from the patient, removing waste, toxins, and excess water from the patient. This cycle is repeated, e.g., multiple times.

There are various types of peritoneal dialysis therapies, including continuous ambulatory peritoneal dialysis ("CAPD"), automated peritoneal dialysis ("APD"), tidal flow dialysis, and continuous flow peritoneal dialysis ("CFPD"). CAPD is a manual dialysis treatment. Here, the patient manually connects an implanted catheter to a drain to allow used or spent dialysis fluid to drain from the peritoneal chamber. The patient then switches fluid communication so that the patient catheter communicates with a bag of fresh dialysis fluid to infuse the fresh dialysis fluid through the catheter and into the patient. The patient disconnects the catheter from the fresh dialysis fluid bag and allows the dialysis fluid to dwell within the peritoneal chamber, wherein the transfer of waste, toxins and excess water takes place. After a dwell period, the patient repeats the manual dialysis procedure, for example, four times per day. Manual peritoneal dialysis requires a significant amount of time and effort from the patient, leaving ample room for improvement.

Automated peritoneal dialysis ("APD") is similar to CAPD in that the dialysis treatment includes drain, fill, and dwell cycles. Automated PD machines, however, perform the cycles automatically, typically while the patient sleeps. The PD machines free patients from having to manually perform the treatment cycles and from having to transport supplies during the day. The PD machines connect fluidly to an implanted catheter, to a source or bag of fresh dialysis fluid and to a fluid drain. The PD machines pump fresh dialysis fluid from a dialysis fluid source, through the catheter and into the patient's peritoneal chamber. The PD machines also allow for the dialysis fluid to dwell within the chamber and for the transfer of waste, toxins, and excess water to take place. The source may include multiple liters of dialysis fluid including several solution bags.

The PD machines pump used or spent dialysate from the patient's peritoneal cavity, though the catheter, to a drain. As with the manual process, several drain, fill, and dwell cycles occur during dialysis. A "last fill" may occur at the end of an APD treatment. The last fill fluid may remain in the peritoneal chamber of the patient until the start of the next treatment, or may be manually emptied at some point during the day.

In any of the above modalities, the automated machine and even manual CAPD operate typically with a disposable set, which is discarded after a single use. Depending on the complexity of the disposable set, the cost of using one set per day may become significant. Also, daily disposables require space for storage, which can become a nuisance for home owners and businesses. Moreover, daily disposable replacement requires daily setup time and effort by the patient or caregiver at home or at a clinic.

For each of the above reasons, it is desirable to provide an APD machine that reduces disposable waste and that does so in an aseptic manner.
US 2022/203005 A1 discloses a peritoneal dialysis ("PD") system including a housing; a dialysis fluid pump housed by the housing and including a reusable pump body that accepts PD fluid for pumping; a dialysis fluid inline heater housed by the housing and including a reusable heater body that accepts PD fluid for heating; at least one reusable PD fluid line extending from the housing; at least one disinfection connector supported by the housing and configured to accept one of the at least one reusable PD fluid line; and a control unit configured to run a disinfection sequence after a PD treatment, wherein each of the at least one reusable PD fluid line is connected to one of the at least one disinfection connectors, and wherein at least one of the dialysis fluid pump or the dialysis fluid inline heater is actuated by the control unit during the disinfection sequence.

### SUMMARY

The present disclosure sets forth an automated peritoneal dialysis ("PD") system that provides improved reusability. The system includes a PD machine or cycler. The PD machine is capable of delivering fresh, heated PD fluid to the patient at, for example, 14 kPa (2.0 psig) or higher. The PD machine is capable of removing used PD fluid or effluent from the patient at, for example, between -5 kPa (-.73 psig) and -15 kPa (-2.2psig), such as -9 kPa (-1.3 psig) or higher. Fresh PD fluid may be delivered via a dual lumen patient line to the patient and is first heated to body fluid temperature, e.g., 37°C. The heated PD fluid is then pumped through a fresh PD fluid lumen of the dual lumen patient line to a disposable filter set, which is connected to the patient's transfer set, which is in turn connected to an indwelling catheter leading into the patient's peritoneal cavity. The disposable filter set communicates fluidly with the fresh and used PD fluid lumens of the dual lumen patient line. The disposable filter set is provided in one embodiment as a last chance filter for the PD machine, which may be heat disinfected between treatments.

The system may include one or more PD fluid container or bag that supplies fresh PD fluid to the PD machine or cycler. The PD machine or cycler may include internal lines having two-way or three-way valves and at least one PD fluid pump for pumping fresh PD fluid from the one or more PD fluid container or bag to a patient and for removing used PD fluid from the patient to a house drain or drain container. One or more flexible PD fluid line leads from the PD machine or cylcer's internal lines to the one or more PD fluid container or bag. The flexible dual lumen patient line mentioned above leads from the PD machine or cylcer's internal lines to the patient. A flexible drain line leads from the PD machine or cylcer's internal lines to the house drain or drain container. The system in one embodiment disinfects all internal lines, the PD fluid lines and the dual lumen patient line after treatment for reuse in the next treatment. The disinfection may involve heat disinfection using leftover fresh PD fluid.

The PD system in one embodiment retracts the reusable dual lumen patient line into a housing of the PD machine during disinfection. To do so, the PD machine may include an internal hose reel that pulls the dual lumen patient line into the housing. Maintaining the dual lumen patient line within the housing during disinfection is advantageous because the patient line is held in a coiled arrangement such that there is less surface area to lose heat. Also, the coiled patient line is retained within the housing, which acts as a thermal insulator relative to ambient temperature and ambient air fluctuations. In short, retracting the patient line into the housing during heat disinfection increases the efficiency of same.

A problem associated with retracting the dual lumen patient line into the housing is that debris collected by the patient line may be pulled into the housing. The debris may contaminate the internal components of the PD machine and possibly migrate into the inside of patient tubing. The PD machine and associated system and method of the present disclosure solves the debris collection problem by providing an external tubing surface cleaning unit that removes debris from the tubing and applies a disinfectant to the tubing as the tubing is pulled into the housing of the PD machine.

The external tubing surface cleaning unit in one embodiment includes a collar having an inner diameter that has a shape that matches an outer diameter shape of the dual lumen patient line. The dual lumen patient line may have a circular or substantially circular outer diameter in which case the collar includes a matching circular inner diameter. Alternatively, the dual lumen patient line may have an oblong or elliptical outer diameter in which case the collar includes a matching oblong or elliptical inner diameter. The inner diameter of the collar may be slightly larger than the outer diameter of the dual lumen patient line.

A foam scrubber is provided along the inner diameter of the collar, such that the foam scrubber extends around and contacts a full 360 degrees of the dual lumen patient line as the patient line is pulled through the external tubing surface cleaning unit of the present disclosure. The foam scrubber accordingly has a cylindrical or tubular portion that is held, e.g., mechanically press-fit and/or adhered, within the collar. The foam scrubber also includes a disinfectant application extension that extends from the cylindrical or tubular portion into a disinfectant reservoir. The disinfectant reservoir holds a disinfectant, such as an environmentally friendly disinfectant, e.g., citric acid or a povidone-iodine solution. The disinfectant application extension of the foam scrubber allows the disinfectant to flow or migrate from the disinfectant reservoir to the foam scrubber cylindrical or tubular portion, where the disinfectant may then be transferred to the exterior surface of the dual lumen patient line as it is pulled through the external tubing surface cleaning unit. The foam scrubber accordingly wipes off the external surface of the patient line and applies disinfectant to same.

The foam scrubber may be made for example from polyurethane flexible foam or silicone foam, e.g., with a shore hardness ranging from Shore A50 to Shore A60, which has good absorption properties when dipped with disinfectant, for example, a povidone-iodine solution or citric acid. The collar and the reservoir are made of plastic in one embodiment, such as, polyvinylchloride ("PVC") or a non-PVC material, such as polyethylene ("PE"), polyurethane ("PU"), polycarbonate ("PC") and/or polyetheretherketone ("PEEK").

The reservoir includes a sealed septum or other type of openable sealed closure that accepts a syringe or other type of disinfectant administration device. The patient or caregiver periodically fills the syringe or other disinfectant administration device with disinfectant, e.g., citric acid or a povidone-iodine solution. The patient then inserts a distal tip of the disinfectant administration device through the openable sealed closure and causes disinfectant to be distributed from the disinfectant administration device into the reservoir to fill the reservoir with disinfectant. Once the filling of the reservoir is complete, the user or caregiver removes the disinfectant administration device from the reservoir, after which the openable sealed closure either self-seals closed or is sealed closed via the patient or caregiver, e.g., applying a threaded cap.

Knowing when to refill the disinfectant reservoir may be accomplished in a plurality of ways. In one embodiment, the disinfectant level is visible to the user, wherein at least a portion of the reservoir is made of a clear or non-opaque material such that the patient or caregiver is able to visually discern when to add disinfectant to the reservoir. Alternatively or additionally, one or more low level sensor is provided with the PD machine. The low level sensor is positioned and arranged adjacent to the reservoir so as to be able to detect when the level of disinfectant has fallen below a refill level, indicating that the reservoir needs to be refilled. The sensor sends a signal to a control unit of the PD machine that is indicative of the low disinfectant level, upon which the control unit may cause a user interface of the PD machine to provide an audio, visual or audiovisual message to the patient or caregiver instructing that the disinfectant reservoir needs to be refilled. The one or more low level sensor may, for example, be a Hall Effect, magnetic, inductive, capacitive or optical sensor.

Alternatively or additionally, the control unit may cooperate with the patient or caregiver to know when to refill the reservoir with disinfectant. In one embodiment, the patient upon refilling the disinfectant reservoir presses or enters a refill confirm button or input into the control unit. The control unit at that point knows that the reservoir is full. The control unit is also programmed to know how much disinfectant is used for each retraction of the dual lumen patient line, i.e., how many disinfection sequence patient line retractions may be performed after the disinfectant reservoir is refilled. When the control unit determines that the current retraction is at or near the refill limit, the control unit causes the user interface of the PD machine to provide an audio, visual or audiovisual message to the patient or caregiver instructing that the disinfectant reservoir needs to be refilled.

The external tubing surface cleaning unit of the present disclosure may be implemented in a plurality of different ways. In a first way, the cleaning unit is provided primarily as an integrated and largely durable or reusable unit. The collar and reservoir are permanent unless damaged and needing replacement, while the foam scrubber may be a semi-permanent component that is replaced periodically. The patient is provided with a reusable syringe or other disinfectant administration device and a supply of disinfectant, such as citric acid or a povidone-iodine solution, which is replaced periodically. The extemal tubing surface cleaning unit is located within the housing of the PD machine, such that it is largely hidden from the patient or caregiver. The septum or other type of openable sealed closure provided at the reservoir is visible and accessible from outside the housing, such that the reservoir may be accessed by the patient or caregiver for disinfectant refill.

In a second way, the external tubing cleaning unit is provided as a detachable and replaceable unit. Here, the collar, reservoir and foam scrubber may be semi-permanent components, wherein the external tubing cleaning unit is removed and replaced as a whole when needed, e.g., when the foam scrubber needs replacement. The septum or other type of openable sealed closure may again be provided at the reservoir so that the patient or caregiver can introduce new disinfectant when needed. The patient is provided with a reusable syringe or other disinfectant administration device and a supply of disinfectant, such as citric acid or a povidone-iodine solution, which is replaced periodically. In an alternative embodiment, openable sealed closure is not provided and the external tubing cleaning unit is instead replaced when the reservoir runs out of disinfectant. In any embodiment, the reservoir of the detachable and replaceable unit may be provided with a tab or other feature sized and shaped to snap-fit into a mating aperture formed in the housing of the PD machine. A portion of the collar may also be used to snap-fit into a mating aperture formed in the housing of the PD machine. Providing multiple snap-fitting engagements helps the external tubing cleaning unit to remain stable while the reusable line, e.g., reusable dual lumen patient line is pulled through the cleaning unit while being scrubbed and disinfected via contact with the foam scrubber.

In a first aspect of the present disclosure, which may be combined with any other aspect, or portion thereof, a peritoneal dialysis ("PD") system includes a housing; a PD fluid pump housed by the housing; a reusable PD fluid line in fluid communication with the PD fluid pump, the reusable PD fluid line positioned and arranged to be pulled into the housing; and an external tubing surface cleaning unit including a scrubber sized and shaped to extend around and contact the reusable PD fluid line to remove debris from at least a portion of an exterior of the reusable PD fluid line as it is pulled into the housing.

In a second aspect of the present disclosure, which may be combined with any other aspect, or portion thereof, the reusable PD fluid line is a reusable patient line.

In a third aspect of the present disclosure, which may be combined with any other aspect, or portion thereof, the reusable patient line is a reusable dual lumen patient line.

In a fourth aspect of the present disclosure, which may be combined with any other aspect, or portion thereof, the PD system includes a hose reel located within the housing, the hose reel configured to pull the reusable PD fluid line into the housing.

In a fifth aspect of the present disclosure, which may be combined with any other aspect, or portion thereof, the PD system includes a control unit configured to cause a disinfection sequence to be performed in which an interior of the reusable PD fluid line is disinfected, and wherein the reusable PD fluid line is pulled into the housing prior to the disinfection sequence being performed.

In a sixth aspect of the present disclosure, which may be combined with any other aspect, or portion thereof, the scrubber is a foam or fibrous scrubber formed so as to apply disinfectant to at least the portion of the exterior of the reusable PD fluid line as it is pulled into the housing.

In a seventh aspect of the present disclosure, which may be combined with any other aspect, or portion thereof, the foam or fibrous scrubber includes a cylindrical portion sized and shaped to extend around and apply disinfectant to the reusable PD fluid line, and a disinfectant application extension extending from the cylindrical portion into a disinfectant reservoir, wherein the disinfectant application extension allows disinfectant from the disinfectant reservoir to flow to the cylindrical portion.

In an eighth aspect of the present disclosure, which may be combined with any other aspect, or portion thereof, the cylindrical portion of the foam or fibrous scrubber is supported by a collar, the disinfectant reservoir extending from the collar.

In a ninth aspect of the present disclosure, which may be combined with any other aspect, or portion thereof, the external tubing surface cleaning unit includes a disinfectant reservoir configured to hold a disinfectant for application to at least the portion of the exterior of the reusable PD fluid line as it is pulled into the housing.

In a tenth aspect of the present disclosure, which may be combined with any other aspect, or portion thereof, the disinfectant reservoir includes an openable sealed closure configured to allow disinfectant to be introduced into the disinfectant reservoir.

In an eleventh aspect of the present disclosure, which may be combined with any other aspect, or portion thereof, the PD system includes a disinfectant administration device, wherein the openable sealed closure is configured to receive a tip of the disinfectant administration device such that the disinfectant administration device is able to introduce disinfectant into the disinfectant reservoir.

In a twelfth aspect of the present disclosure, which may be combined with any other aspect, or portion thereof, the PD system includes a source of disinfectant and a tube leading from the source of disinfectant to the disinfectant reservoir.

In a thirteenth aspect of the present disclosure, which may be combined with any other aspect, or portion thereof, the external tubing surface cleaning unit is located within the housing.

In a fourteenth aspect of the present disclosure, which may be combined with any other aspect, or portion thereof, the housing provides an opening allowing an openable sealed closure of the external tubing surface cleaning unit to be accessed for refilling disinfectant.

In a fifteenth aspect of the present disclosure, which may be combined with any other aspect, or portion thereof, the external tubing surface cleaning unit is attachable to and detachable from the housing.

In a sixteenth aspect of the present disclosure, which may be combined with any other aspect, or portion thereof, the housing defines at least one mating aperture, and wherein the external tubing surface cleaning unit includes at least one feature sized and shaped to snap-fit within the at least one mating aperture.

In a seventeenth aspect of the present disclosure, which may be combined with any other aspect, or portion thereof, the external tubing surface cleaning unit includes a disinfectant reservoir, and wherein the system includes at least one sensor positioned and arranged to detect a level of disinfectant within the disinfectant reservoir.

In an eighteenth aspect of the present disclosure, which may be combined with any other aspect, or portion thereof, the PD system includes a control unit and a user interface, the at least one sensor outputting to the control unit, and wherein the control unit is configured to cause the user interface to prompt for a disinfectant refill when an output from the at least one sensor indicates a low disinfectant level within the disinfectant reservoir.

In a nineteenth aspect of the present disclosure, which may be combined with any other aspect, or portion thereof, the PD system includes at least one ultraviolet ("UV") light positioned and arranged to disinfect the exterior of the reusable PD fluid line as it is pulled into the housing.

In a twentieth aspect of the present disclosure, which may be combined with any other aspect, or portion thereof, the PD system includes a source of disinfectant and a tube leading from the source of disinfectant into the external tubing surface cleaning unit so as to be able to apply disinfectant to at least the portion of the exterior of the reusable PD fluid line as it is pulled into the housing.

In a twenty-first aspect of the present disclosure, which may be combined with any other aspect, or portion thereof, a peritoneal dialysis ("PD") system includes a PD machine including a housing; a reusable PD fluid line positioned and arranged to be pulled into the housing; and an external tubing surface cleaning unit including a scrubber sized and shaped to extend around and contact the reusable PD fluid line to remove debris from at least a portion of an exterior of the reusable PD fluid line as it is pulled into the housing.

In a twenty-second aspect of the present disclosure, which may be combined with any other aspect, or portion thereof, a method for cleaning an exterior of a reusable PD fluid line comprising: enabling a reusable PD fluid line to be retracted into a PD fluid machine; and causing debris from at least a portion of an exterior of the reusable PD fluid line to be removed as the reusable PD fluid line is retracted into the PD fluid machine using a scrubber sized and shaped to extend around and contact the reusable PD fluid line.

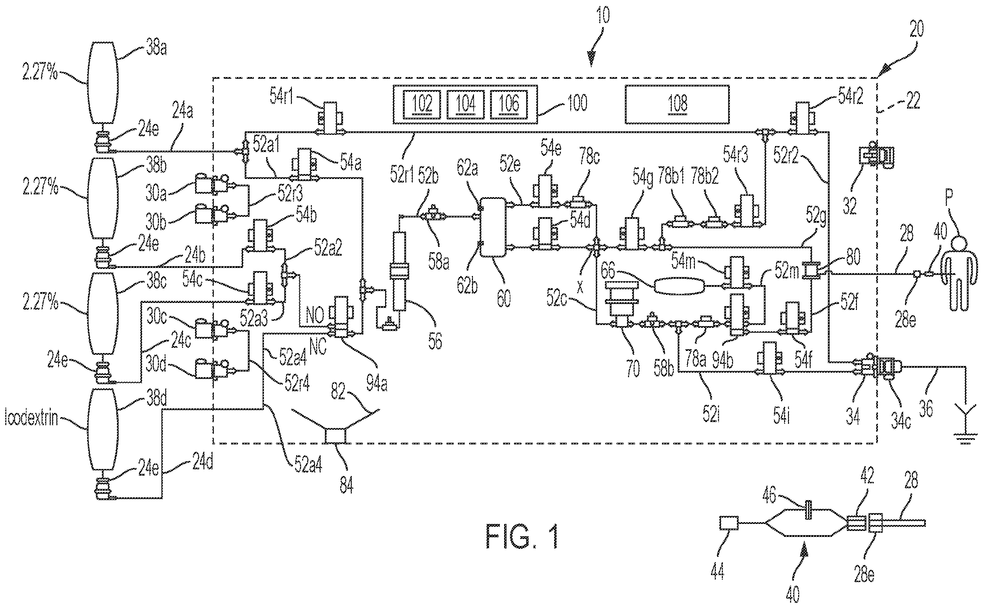

Any of the features, functionality and alternatives described in connection with any one or more of Figs. 1 to 9 may be combined with any of the features, functionality and alternatives described in connection with any other of Figs. 1 to 9.

In light of the above aspects and present disclosure set forth herein, it is an advantage of the present disclosure to provide a dialysis system and a method that reduce disposable waste in an aseptic manner.

It is another advantage of the present disclosure to provide a dialysis system and a method that help to prevent debris and other matter from entering a dialysis machine.

It is a further advantage of the present disclosure to provide a PD system that is easy to reload with a disinfectant.

It is yet another advantage of the present disclosure to provide a dialysis system and a method that help to reduce service and replacement of a reusable tube.

It is a yet a further advantage of the present disclosure to provide a PD system and a method that increase safety associated with reusable tubing.

Additional features and advantages are described in, and will be apparent from, the following Detailed Description and the Figures. The features and advantages described herein are not all-inclusive and, in particular, many additional features and advantages will be apparent to one of ordinary skill in the art in view of the figures and description. Also, any particular embodiment does not have to have all of the improvements or advantages listed herein. In particular, the system of the present disclosure may have any one or more or all of the drip prevention structure and methodology, PD fluid container emptying structure and methodology and patient connection before drain check structure and methodology described herein. Moreover, it should be noted that the language used in the specification has been selected principally for readability and instructional purposes.

### BRIEF DESCRIPTION OF THE FIGURES

Fig. 1 is a fluid flow schematic of one embodiment for a medical fluid system, e.g., a PD fluid system, that is set for treatment.
Fig. 2 is a fluid flow schematic of one embodiment for a medical fluid system, e.g., PD fluid system, that is set for disinfection.
Fig. 3 is a perspective view illustrating one embodiment for a housing of a PD machine and a retractable and reusable PD fluid patient line of the present disclosure.
Fig. 4 is a front elevation view of one embodiment for an external tubing surface cleaning unit of the present disclosure.
Fig. 5 is a side elevation view of one embodiment for an external tubing surface cleaning unit of the present disclosure shown mounted within the PD machine, wherein the reusable line, e.g., reusable patient line, is partially retracted within the PD machine.
Fig. 6 is a side elevation view of one embodiment for an external tubing surface cleaning unit of the present disclosure shown mounted within the PD machine, wherein the reusable line, e.g., reusable patient line, is fully retracted within the PD machine.
Fig. 7 is a front perspective view of an attachable, detachable and replaceable external tubing surface cleaning unit of the present disclosure.
Fig. 8 is a rear perspective view of an attachable, detachable and replaceable external tubing surface cleaning unit of the present disclosure.
Fig. 9 is a front perspective view of an attachable, detachable and replaceable external tubing surface cleaning unit of the present disclosure abutted against a PD machine for operation.

### DETAILED DESCRIPTION

### System Overview

Referring now to the drawings and in particular to Fig. 1, a medical system having the external tubing surface disinfection of the present disclosure is illustrated via peritoneal dialysis ("PD") system 10. System 10 includes a PD machine or cycler 20 and a control unit 100 having one or more processor 102, one or more memory 104, video controller 106 and user interface 108. User interface 108 may alternatively or additionally be a remote user interface, e.g., via a tablet or smartphone. Control unit 100 may also include a transceiver and a wired or wireless connection to a network (not illustrated), e.g., the internet, for sending treatment data to and receiving prescription instructions/changes from a doctor's or clinician's server interfacing with a doctor's or clinician's computer. Control unit 100 in an embodiment controls all electrical fluid flow and heating components of system 10 and receives outputs from all sensors of system 10. System 10 in the illustrated embodiment includes durable and reusable components that contact fresh and used PD fluid, which necessitates that PD machine or cycler 20 be disinfected between treatments, e.g., via heat disinfection.

System 10 in Fig. 1 includes an inline resistive heater 56, reusable supply lines or tubes 52a1 to 52a4 and 52b, air trap 60 operating with respective upper and lower level sensors 62a and 62b, air trap valve 54d, vent valve 54e located along vent line 52e, reusable line or tubing 52c, PD fluid pump 70, temperature sensors 58a and 58b, pressure sensors 78a, 78b1, 78b2 and 78c, reusable patient tubing or lines 52f and 52g having respective valves 54f and 54g, reusable dual lumen patient line 28, a spring-actuated hose reel 80 for retracting patient line 28, reusable drain tubing or line 52i extending to drain line connector 34 and having a drain line valve 54i, and reusable recirculation tubing or lines 52r1 and 52r2 operating with respective disinfection valves 54r1 and 54r2. A third recirculation or disinfection tubing or line 52r3 extends between disinfection or PD fluid line connectors 30a and 30b for use during disinfection. A fourth recirculation or disinfection tubing or line 52r4 extends between disinfection connectors 30c and 30d for use during disinfection.

System 10 further includes PD fluid containers or bags 38a to 38c (e.g., holding the same or different formulations of PD fluid), which connect to distal ends 24e of reusable PD fluid lines 24a to 24c, respectively. System 10d further includes a fourth PD fluid container or bag 38d that connects to a distal end 24e of reusable PD fluid line 24d. Fourth PD fluid container or bag 38d may hold the same or different type (e.g., icodextrin) of PD fluid than provided in PD fluid containers or bags 38a to 38c. Reusable PD fluid lines 24a to 24d extend in one embodiment through apertures (not illustrated) defined or provided by housing 22 of cycler 20.

System 10 in the illustrated embodiment includes four disinfection or PD fluid line connectors 30a to 30d for connecting to distal ends 24e of reusable PD fluid lines 24a to 24d, respectively, during disinfection. System 10 also provides a patient line connector 32 that includes an internal lumen, e.g., a U-shaped lumen, which for disinfection directs fresh or used dialysis fluid from one PD fluid lumen of a connected distal end 28e of reusable dual lumen patient line 28 into the other PD fluid lumen. Reusable supply tubing or lines 52a1 to 52a4 communicate with reusable supply lines 24a to 24d, respectively. Reusable supply tubing or lines 52a1 to 52a3 operate with valves 54a to 54c, respectively, to allow PD fluid from a desired PD fluid container or bag 38a to 38c to be pulled into cycler 20. Three-way valve 94a in the illustrated example allows for control unit 100 to select between (i) 2.27% (or other) glucose dialysis fluid from container or bag 38b or 38c and (ii) icodextrin from container or bag 38d. In the illustrated embodiment, icodextrin from container or bag 38d is connected to the normally closed port of three-way valve 94a.

System 10 is constructed in one embodiment such that drain line 52i during a patient fill is fluidly connected downstream from PD fluid pump 70. In this manner, if drain valve 54i fails or somehow leaks during the patient fill of patient P, fresh PD fluid is pushed down disposable drain line 36 instead of used PD fluid potentially being pulled into pump 70. Disposable drain line 36 is in one embodiment removed for disinfection, wherein drain line connector 34 is capped via a cap 34c to form a closed disinfection loop. PD fluid pump 70 may be an inherently accurate pump, such as a piston pump, or less accurate pump, such as a gear pump that operates in cooperation with a flowmeter (not illustrated) to control fresh and used PD fluid flowrate and volume.

System 10 may further include a leak detection pan 82 located at the bottom of housing 22 of cycler 20 and a corresponding leak detection sensor 84 outputting to control unit 100. In the illustrated example, system 10 is provided with an additional pressure sensor 78c located upstream of PD fluid pump 70, which allows for the measurement of the suction pressure of pump 70 to help control unit 100 more accurately determine pump volume. Additional pressure sensor 78c in the illustrated embodiment is located along vent line 52e, which may be filled with air or a mixture of air and PD fluid, but which should nevertheless be at the same negative pressure as PD fluid located within PD fluid line 52c.

System 10 in the example of Fig. 1 includes redundant pressure sensors 78b1 and 78b2, the output of one of which is used for pump control, as discussed herein, while the output of the other pressure sensor is a safety or watchdog output to make sure the control pressure sensor is reading accurately. Pressure sensors 78b1 and 78b2 are located along a line including a third recirculation valve 54r3. System 10 may further employ one or more cross, marked via an X in Fig. 1, which may (i) reduce the overall amount and volume of the internal, reusable tubing, (ii) reduce the number of valves needed, and (iii) allow the portion of the fluid circuitry shared by both fresh and used PD fluid to be minimized.

System 10 in the example of Fig. 1 further includes a source of acid, such as a citric acid container or bag 66. Citric acid container or bag 66 is in selective fluid communication with second three-way valve 94b via a citric acid valve 54m located along a citric acid line 52m. Citric acid line 52m is connected in one embodiment to the normally closed port of second three-way valve 94b, so as to provide redundant valves between disinfectant container or bag 66 and the PD fluid circuit during treatment. The redundant valves ensure that no citric acid reaches the treatment fluid lines during treatment. Citric acid is used instead during disinfection.

Control unit 100 in an embodiment uses feedback from any one or more of pressure sensors 78a to 78c to enable PD machine 20 to deliver fresh, heated PD fluid to the patient at, for example, 14 kPa (2.0 psig) or higher. The pressure feedback is used to enable PD machine 20 to remove used PD fluid or effluent from the patient at, for example, between -5 kPa (-.73 psig) and -15 kPa (-2.2psig), such as -9 kPa (-1.3 psig) or higher (more negative). The pressure feedback may be used in a proportional, integral, derivative ("PID") pressure routine for pumping fresh and used PD fluid at a desired positive or negative pressure.

Inline resistive heater 56 under control of control unit 100 is capable of heating fresh PD fluid to body temperature, e.g., 37°C, for delivery to patient P at a desired flowrate. Control unit 100 in an embodiment uses feedback from temperature sensor 58a in a PID temperature routine for pumping fresh PD fluid to patient P at a desired temperature. The control and operation of inline resistive heater 56 for heat disinfection is discussed in detail below.

Fig. 1 also illustrates that system 10 includes and uses a disposable filter set 40, which communicates fluidly with the fresh and used PD fluid lumens of dual lumen patient line 28. Disposable filter set 40 includes a disposable connector 42 that connects to a distal end 28e of reusable patient line 28. Disposable filter set 40 also includes a connector 44 that connects to the patient's transfer set. Disposable filter set 40 further includes a sterilizing grade filter membrane 46 that further filters fresh PD fluid. Disposable filter set 40 is provided in one embodiment as a last chance filter for PD machine 20, which has been heat disinfected between treatments. Any pathogens that may remain after disinfection, albeit unlikely, are filtered from the PD fluid via the sterilizing grade filter membrane 46 of disposable filter set 40.

Fig. 1 illustrates system 10 setup for treatment with PD fluid containers or bags 38a to 38d connected via reusable, flexible PD fluid lines 24a to 24d, respectively. Dual lumen patient line 28 is connected to patient P via disposable filter set 40. Disposable drain line 36 is connected to drain line connector 34. In Fig. 1, PD machine or cycler 20 of system 10 is configured to perform multiple patient drains, patient fills, patient dwells, and a priming procedure, as part of or in preparation for treatment.

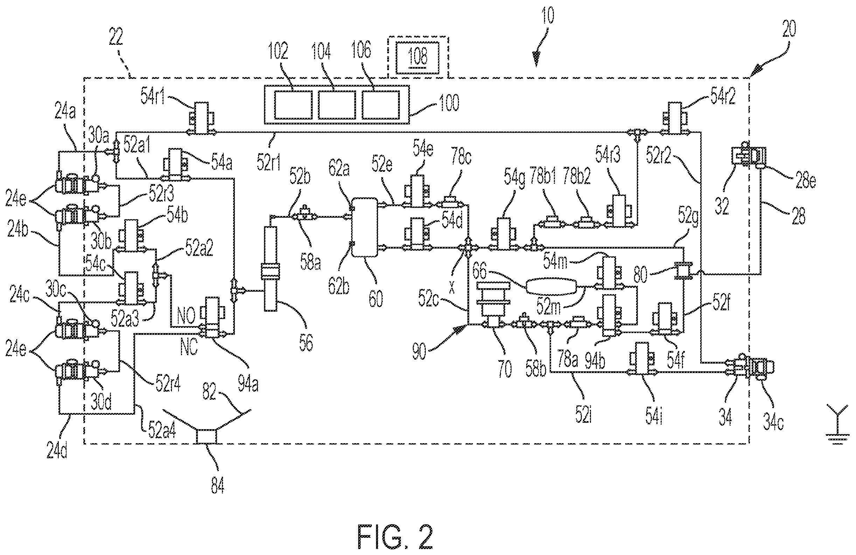

Fig. 2 illustrates system 10 in a disinfection mode. PD fluid containers or bags 38a to 38d are removed and flexible PD fluid lines 24a to 24d are plugged instead in a sealed manner into disinfection or PD fluid line connectors 30a to 30d, respectively. Reusable dual lumen patient line 28 is disconnected from disposable filter set 40 (which is discarded), and distal end 28e of dual lumen patient line 28 is plugged sealingly into patient line connector 32. Disposable drain line 36 is removed from drain line connector 34 and discarded. Drain line connector 34 is capped via cap 34c to form a closed disinfection loop 90. PD machine or cycler 20 of system 10 in Fig. 2 is configured to perform a disinfection sequence, e.g., a heat disinfection sequence in which fresh PD fluid is heated via inline heater 56 to a disinfection temperature of, e.g., 75°C or greater. PD fluid pump 70 circulates the heated PD fluid closed disinfection loop 90 for an amount of time needed to properly disinfect the fluid components and lines of the disinfection loop.

### External Tubing Surface Disinfection

Figs. 1 and 2 illustrate hose reel 80 for retracting dual lumen patient line 28 into housing 22 of PD machine 20 when desired. PD system 10 in one embodiment retracts reusable patient line 28 into housing 22 during disinfection, e.g., after treatment when patient P disconnects from filter set 40 and removes the filter set from the end of patient line 28. Internal hose reel 80 is in one embodiment spring-actuated to automatically pull dual lumen patient line 28 into housing 22 after treatment and before disinfection begins. Maintaining dual lumen patient line 28 within housing 22 during disinfection is advantageous because patient line 22 is held in a coiled arrangement such that there is less surface area to lose heat. Also, the coiled patient line 28 is retained within housing 22, which acts as a thermal insulator relative to ambient temperature and ambient air fluctuations. In short, retracting patient line 28 into housing 22 during heat disinfection increases the efficiency of same.

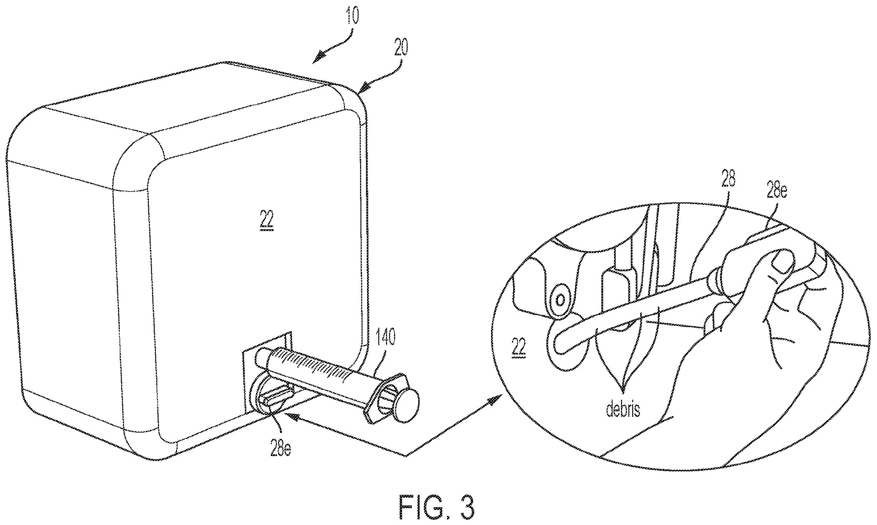

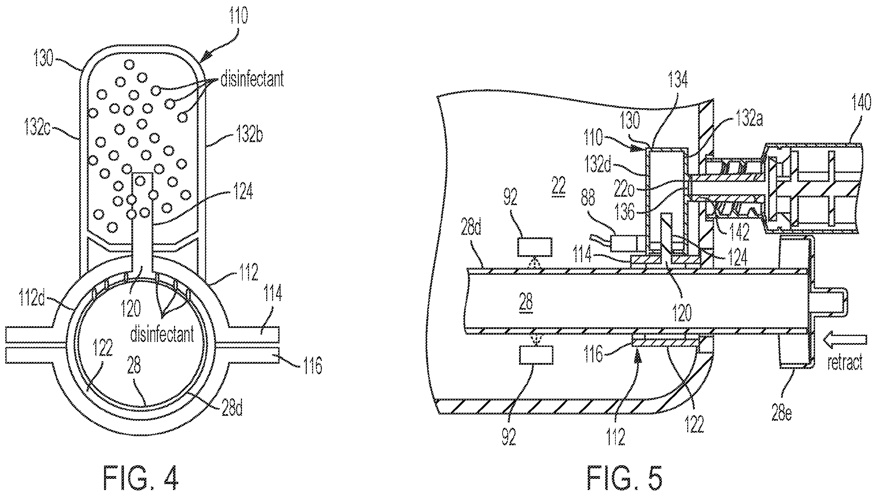

Fig. 3 illustrates that a problem associated with retracting dual lumen patient line 28 into housing 22 of PD machine 20 of system 10 is that debris collected by the patient line may be pulled into the housing. The debris may contaminate the internal components (see Figs. 1 and 2) of PD machine 20 and possibly migrate into the inside of patient tubing 28. Fig. 3 further illustrates a syringe or other disinfectant administration device 140 for refilling disinfectant. As illustrated in Figs. 4 to 9, PD machine 20 and associated system 10 and method of the present disclosure solves the debris collection problem by providing an external tubing surface cleaning unit 110 that removes debris from reusable tubing, such as dual lumen patient line 28, and applies a disinfectant to the tubing as the tubing is pulled into housing 22 of PD machine 20 via spring-actuated hose reel 80.

Figs. 4 to 8 illustrate that external tubing surface cleaning unit 110 in one embodiment includes a collar 112. Collar 112 in the illustrated embodiment is formed from a first, e.g., upper, half 114 and a second, e.g., lower, half 116. First half 114 and second half 116 form an inner diameter 112d that has a shape that matches the shape of an outer diameter 28d of dual lumen patient line 28. Dual lumen patient line 28 may have a circular or substantially circular outer diameter in which case halves 114, 116 of collar 112 form a matching circular inner diameter 112d. Alternatively, dual lumen patient line 28 may have an oblong or elliptical outer diameter 28d in which case halves 114, 116 of collar 112 form a matching oblong or elliptical inner diameter 112d. Inner diameter 112d of collar 112 may be slightly larger than outer diameter 28d of dual lumen patient line 28.

Figs. 4 to 8 also illustrate that a foam scrubber 120 is provided along the inner diameter of halves 114, 116 of collar 112, such that foam scrubber 120 extends around and contacts a full 360 degrees of dual lumen patient line 28 as the patient line is pulled through external tubing surface cleaning unit 110 of the present disclosure. Foam scrubber 120 in the illustrated embodiment has a cylindrical or tubular portion 122 that is held, e.g., mechanically press-fit and/or adhered, within halves 114, 116 of collar 112. Foam scrubber 120 also includes a disinfectant application extension 124 that extends from cylindrical or tubular portion 122 into a disinfectant reservoir 130.

Disinfectant reservoir 130 in the illustrated embodiment includes flat sides 132a to 132d and a rounded top 134. A bottom of disinfectant reservoir 130 in the illustrated embodiment is formed by first or upper half 114 of collar 112. Disinfectant reservoir 130 holds a disinfectant, such as an environmentally friendly disinfectant, e.g., citric acid or a povidone-iodine solution. Disinfectant application extension 124 of foam scrubber 120 allows the disinfectant to flow or migrate from disinfectant reservoir 130 to foam scrubber cylindrical or tubular portion 122, where the disinfectant may then be transferred to the exterior surface 28d of dual lumen patient line 28 as it is pulled through external tubing surface cleaning unit 110. Foam scrubber 120 accordingly wipes off the external surface or diameter 28d of patient line 28 and applies disinfectant to same.

Foam scrubber 120 may be made for example from polyurethane flexible foam or silicone foam, e.g., with a shore hardness ranging from Shore A50 to Shore A60, which has good absorption properties when dipped with disinfectant, for example, a povidone-iodine solution or citric acid.. Collar 112 and disinfectant reservoir 130 are made of, e.g., molded from, one or more plastic in one embodiment, such as, polyvinylchloride ("PVC") or a non-PVC material, such as polyethylene ("PE"), polyurethane ("PU"), polycarbonate ("PC") and/or polyetheretherketone ("PEEK").

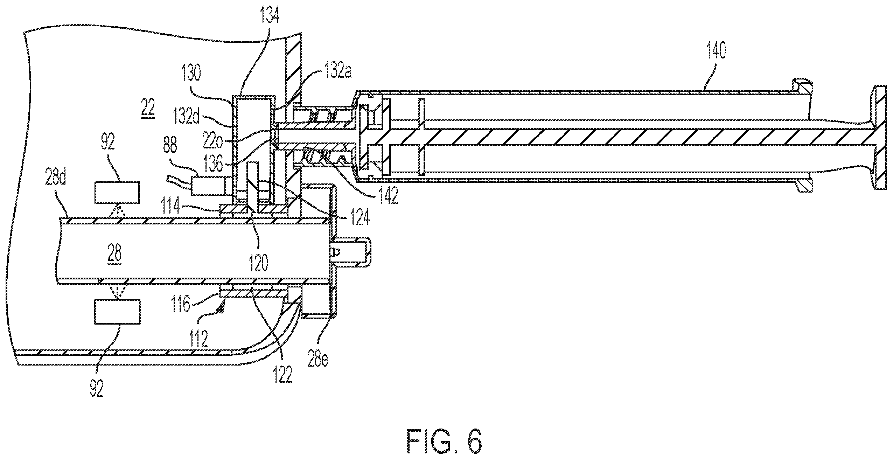

Figs. 5 to 7 illustrate that in one embodiment, flat side 132a of reservoir 130 includes a sealed septum or other type of openable sealed closure 136 that accepts the tip 142 (Figs. 5, 6) of a syringe 140 or other type of disinfectant administration device. The patient or caregiver periodically fills the syringe or other disinfectant administration device 140 with disinfectant, e.g., citric acid or a povidone-iodine solution. The patient then inserts distal tip 142 of disinfectant administration device 140 through the openable sealed closure 136 and causes disinfectant to be distributed from disinfectant administration device 140 into disinfectant reservoir 130 to fill the reservoir with disinfectant, e.g., citric acid or a povidone-iodine solution. Once the filling of reservoir 130 is complete, the user or caregiver removes disinfectant administration device 140 from reservoir 130, after which the openable sealed closure 136 either self-seals closed or is sealed closed via the patient or caregiver, e.g., applying a threaded cap.

Knowing when to refill disinfectant reservoir 130 may be accomplished in a plurality of ways. In one embodiment, the disinfectant level is visible to the user, wherein at least one side 132a to 132d of reservoir 130 is made of a clear or non-opaque material such that the patient or caregiver is able to visually discern when to add disinfectant to the reservoir. Alternatively or additionally, one or more low level sensor 88 outputting to control unit 100 is provided with PD machine 20. Low level sensor 88 as illustrated in Figs. 5 and 6 is positioned and arranged adjacent to disinfectant reservoir 130 so as to be able to detect when the level of disinfectant has fallen below a refill level, indicating that the reservoir needs to be refilled. One or more level sensor 88 sends a corresponding signal to control unit 100 that is indicative of the low disinfectant level, upon which control unit 100 cause user interface 108 to provide an audio, visual or audiovisual message to the patient or caregiver instructing that the disinfectant reservoir needs to be refilled. User interface 108 may also provide a refill confirm button or selector that the patient or caregiver needs to press after refilling the disinfectant, e.g., in order for a next treatment to be performed. One or more low level sensor 88 may, for example, be a Hall effect, magnetic, inductive, capacitive or optical sensor.

Alternatively or additionally, control unit 100 may cooperate with the patient or caregiver to know when to refill reservoir 130 with disinfectant. In one embodiment, the patient upon refilling disinfectant reservoir 130 presses or enters a refill confirm button or input into user interface 108 of control unit 100. Control unit 100 at that point knows that reservoir 130 is full. Control unit 100 is also programmed to know how much disinfectant is used for each retraction of dual lumen patient line 28, i.e., how many disinfection sequence patient line retractions may be performed after disinfectant reservoir 130 is refilled. When control unit 100 determines that the current retraction is at or near the refill limit, control unit 28 causes user interface 10 to provide an audio, visual or audiovisual message to the patient or caregiver instructing that the disinfectant reservoir needs to be refilled.

Figs. 5 and 6 further illustrate that in one embodiment, one or more ultraviolet ("UV") light 92 is provided to disinfect the outside of dual lumen patient line 28 in addition to the disinfection provided by the liquid disinfectant. In an embodiment, control unit 100 energizes UV light 92 automatically upon a sensor (not illustrated), such as a proximity, optical or magnetic sensor, sensing the rotation of hose reel 80, and indicating same to the control unit. Here, UV light 92 is energized automatically while reusable patient line 28 is retracted into housing 22 of PD machine 20. UV light kills pathogens located on the outside of reusable patient line 28. It may thus be possible to keep scrubber 120 of cleaning unit 110 for the purpose of wiping off the outside of reusable patient line 28, while removing the liquid disinfectant from the cleaning unit and instead relying on UV light disinfection.

External tubing surface cleaning unit 110 of the present disclosure may be implemented in a plurality of different ways. In a first way as illustrated in Figs. 3, 5 and 6, cleaning unit 110 is provided primarily as an integrated and largely durable or reusable unit. Collar 112 and reservoir are permanent unless damaged and needing replacement, while foam scrubber 120 may be a semi-permanent component that is replaced periodically. The patient is provided with reusable syringe or other disinfectant administration device 140 and a supply of disinfectant (not illustrated), such as bag or jug of citric acid or povidone-iodine solution, which is replaced periodically.

As illustrated in Figs. 3, 5 and 6, external tubing surface cleaning unit 110 in the integrated embodiment is located within housing 22 of PD machine 20, such that it is largely hidden from the patient or caregiver. Cleaning unit 110 may for example be bolted to and/or snap-fitted to the inside of housing 22. As illustrated in Figs. 5 and 6, housing 22 provides an opening 22o, such that septum or other type of openable sealed closure 136 provided at side 132a of reservoir 132 is visible and accessible from the outside of housing 22, such that the reservoir may be accessed by the patient or caregiver for disinfectant refill.

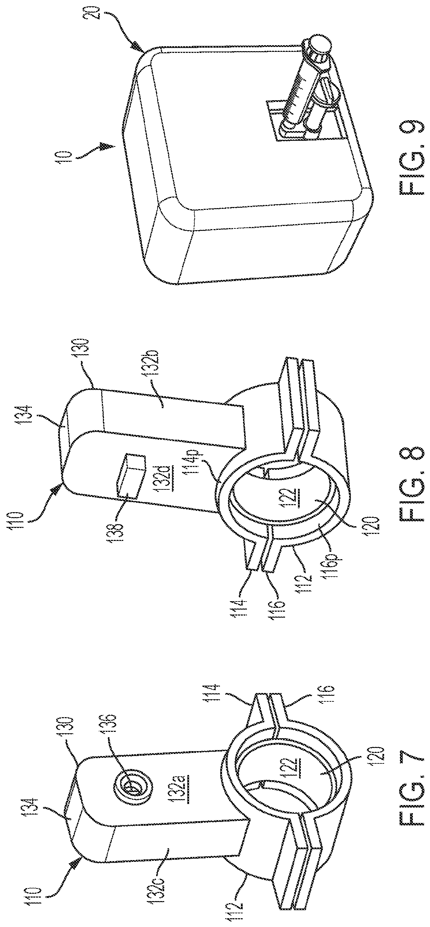

As illustrated in Figs. 7 to 9, in a second way, external tubing cleaning unit 110 is provided as an attachable, detachable and replaceable unit. Here, collar 112, disinfectant reservoir 130 and foam scrubber 120 may be semi-permanent components, wherein external tubing cleaning unit 110 is removed and replaced as a whole when needed, e.g., when foam scrubber 120 needs replacement. Septum or other type of openable sealed closure 136 may again be provided at side 132a of disinfectant reservoir 130 so that the patient or caregiver can introduce new disinfectant when needed. The patient may again be provided with a reusable syringe or other disinfectant administration device 140 and a supply of disinfectant (not illustrated), such as citric acid or a povidone-iodine solution, which is replaced periodically. In an alternative embodiment, openable sealed closure 136 is not provided and external tubing cleaning unit 110 is instead replaced when disinfectant reservoir 130 runs out of disinfectant.

As illustrated in Fig. 8, any version of detachable and replaceable cleaning unit 110 may include a tab or other feature 138 provided at reservoir 130, e.g., at flat side 132d of the reservoir. Tab or other feature 138 is sized and shaped to snap-fit into a mating aperture (not illustrated) formed in housing 22 of the PD machine. Fig. 8 also illustrates that portions 114p, 116p of halves 114, 116 of collar 112 may also be used to snap-fit into a mating aperture (not illustrated) formed in housing 22 of PD machine 20. Providing multiple snap-fitting engagements (e.g., via tab or other feature 138 and collar portions 114p, 116p) helps external tubing cleaning unit 110 to remain stable as the reusable line, e.g., reusable dual lumen patient line 28 is pulled through cleaning unit 110 while being scrubbed and disinfected via contact with foam scrubber 120.

### Alternative Embodiments

In various alternative embodiments, while system 10 is described above with pulling reusable patient line 28 through external tubing cleaning unit 110 automatically via spring-actuated hose reel 80, a spring-actuated hose reel 80 is not required. Instead, a hose reel that is manually operated, e.g., via a rotating handle located outside of housing 22, may be provided instead. Here, reusable patient line 28 is pulled through external tubing cleaning unit 110 manually. Control unit 100 may cause user interface 108 to prompt the patient or caregiver to manually retract reusable patient line 28 into housing 22 after treatment and prior to disinfection.

Further alternatively, while housing 22 of PD machine 20 is illustrated as providing an opening 22o, such that the user may access openable sealed closure 136 of reservoir 130 for disinfectant refill, reservoir 130 is instead refilled from a disinfectant source, e.g., citric acid container or bag 66 (used also during disinfection) located within housing 22 as illustrated in Figs. 1 and 2. Here, a tube (not illustrated) such as a flexible plastic tube may run from disinfectant container or bag 66 to the top of reservoir 130. Disinfectant container or bag 66 may be located elevationally above reservoir 130 such that the disinfectant gravity flows into reservoir 130. The head pressure of disinfectant container or bag 66 may also drive disinfectant into reservoir 130. A sensor, such as any type described herein for low level sensor 88, may be provided as a low level sensor at disinfectant container or bag 66, which outputs to control unit 100. Control unit 100 accordingly knows when the disinfectant within container or bag 66 is low and causes user interface 108 to audibly, visually or audiovisually prompt the patient or caregiver to install a new disinfectant container or bag 66.

In a further alternative embodiment, reservoir 130 is not filled with disinfectant and instead the tube (not illustrated) from container or bag 66 extends into reservoir 130 and terminates just above scrubber 120 so as to be able to dispense disinfectant onto scrubber 120, e.g., just prior to the retraction of reusable patient line 28 through the scrubber. Here, a valve (not illustrated) may be be provided along the tube. Control unit 100 causes the valve to open, e.g., after treatment and prior to the patient line retraction, wherein the valve remains open for only a period of time enough for tubular portion 122 of scrubber 120 to become fully wetted or saturated with disinfectant. If it is determined to be needed, control unit 100 may also open the valve during the retraction of reusable patient line 28 through scrubber 120.

In yet another alternative embodiment, the valve just described is replaced with a clamp. The clamp is in one embodiment normally spring-biased closed. A lever is provided, which actuates the spring (compresses or stretches the spring), allowing the tube extending from disinfectant container or bag 66 or bag to open so that the disinfectant is dispensed onto scrubber. The lever is actuated via a button provided at housing 22 of PD machine 20. When the user releases the button, the spring pushes the button back to its normal unactuated position and causes the clamp to occlude the tube extending from disinfectant container or bag 66, shutting off the flow of the disinfectant. Control unit 100 is in one embodiment programmed to cause user interface 108 to audibly, visually or audiovisually prompt the patient or caregiver to press the button before and/or during retraction of reusable patient line 28.

In yet a further alternative embodiment, the external tubing cleaning unit 110 is located on the outside of housing 22 of PD machine 20 where it is easy to load disinfectant into reservoir 130. Tubular portion 122 of foam scrubber is 120 provided as two halves, one each connected to first half 114 and second half 116 of collar 112. The collar and scrubber halves are normally spring-biased to not contact reusable patient line 28. One or more button is provided to allow the patient or caregiver to force the halves of foam scrubber 120 against the spring-bias and into contact with reusable patient line 28 as it is being retracted into housing 22. When the patient releases the one or more button, the one or more spring is released so as to bias the halves of foam scrubber 120 away from reusable patient line 28. Control unit 100 is in one embodiment programmed to cause user interface 108 to audibly, visually or audiovisually prompt the patient or caregiver to press or otherwise actuate the one or more button before and/or during retraction of reusable patient line 28.

In still a further alternative embodiment, external tubing cleaning unit 110 is provided as a separate handheld unit, which is again configured for easy disinfectant loading. Here, the handheld tubing cleaning unit 110 may be provided as a clamshell with reservoir 130 provided in hinged halves which are each filled with disinfectant. Foam scrubber 120 is also provided in halves, one each for the halves of reservoir 130. Where each half of scrubber 120 is attached to a half of reservoir 130, the reservoir is provided with holes or perforations for allowing the liquid disinfectant to fully wet or saturate the foam scrubber material. Control unit 100 in an embodiment causes user interfsace 108 to audibly, visually or audiovisually prompt the patient or caregiver to place handheld external tubing cleaning unit 110 around reusable patient line 28, directly adjacent to housing 22, as the patient or caregiver initiates the spring-actuated retraction of the patient line via hose reel 80.

It should be understood that other various changes and modifications to the presently preferred embodiments described herein will be apparent to those skilled in the art. The invention is defined by the appended claims. For example, while system 10 is described as cleaning and disinfecting the external surface of dual lumen patient line 28, system 10 may clean and disinfect the external surface of any tube that is retracted into housing 22 of PD machine 20. For example, system 10 may clean and disinfect the external surface of any one or more of reusable PD fluid lines 24a to 24d if pulled into housing 22. System 10 may clean and disinfect the external surface of drain line 36 if instead made to be reusable and pulled into housing 22. Also, while scrubber 120 is described as being a foam scrubber, scrubber 120 could alternatively be made of a fabric or fibrous material that allows disinfectant to flow through the scrubber.

## Claims

1. A peritoneal dialysis ("PD") system (10) comprising:
a housing (22);
a PD fluid pump (70) housed by the housing (22);
a reusable PD fluid line (24a to 24d) in fluid communication with the PD fluid pump (70), the reusable PD fluid line (24a to 24d) positioned and arranged to be pulled into the housing (22); and
an external tubing surface cleaning unit (110) including a scrubber (120) sized and shaped to extend around and contact the reusable PD fluid line (24a to 24d) to remove debris from at least a portion of an exterior (28d) of the reusable PD fluid line (24a to 24d) as it is pulled into the housing (22).

2. The PD system (10) of Claim 1, wherein the reusable PD fluid line (24a to 24d) is a reusable patient line (28).

3. The PD system (10) of Claim 2, wherein the reusable patient line (28) is a reusable dual lumen patient line (28).

4. The PD system (10) of Claim 1, which includes a hose reel (80) located within the housing (22), the hose reel (80) configured to pull the reusable PD fluid line (24a to 24d) into the housing (22).

5. The PD system (10) of Claim 1, which includes a control unit (100) configured to cause a disinfection sequence to be performed in which an interior of the reusable PD fluid line (24a to 24d) is disinfected, and wherein the reusable PD fluid line (24a to 24d) is pulled into the housing (22) prior to the disinfection sequence being performed.

6. The PD system (10) of Claim 1, wherein the scrubber (120) is a foam or fibrous scrubber formed so as to apply disinfectant to at least the portion of the exterior (28d) of the reusable PD fluid line (24a to 24d) as it is pulled into the housing (22).

7. The PD system (10) of Claim 6, wherein the foam or fibrous scrubber (120) includes a cylindrical portion (122) sized and shaped to extend around and apply disinfectant to the reusable PD fluid line (24a to 24d), and a disinfectant application extension (124) extending from the cylindrical portion (122) into a disinfectant reservoir (130), wherein the disinfectant application extension (124) allows disinfectant from the disinfectant reservoir (130) to flow to the cylindrical portion (122).

8. The PD system (10) of Claim 7, wherein the cylindrical portion (122) of the foam or fibrous scrubber (120) is supported by a collar (112), the disinfectant reservoir (130) extending from the collar (112).

9. The PD system (10) of Claim 6, wherein the external tubing surface cleaning unit (110) includes a disinfectant reservoir (130) configured to hold a disinfectant for application to at least the portion of the exterior (28d) of the reusable PD fluid line (24a to 24d) as it is pulled into the housing (22).

10. The PD system (10) of Claim 9, wherein the disinfectant reservoir (130) includes an openable sealed closure (136) configured to allow disinfectant to be introduced into the disinfectant reservoir (130).

11. The PD system (10) of Claim 9, which includes a disinfectant administration device (140), wherein the openable sealed closure (136) is configured to receive a tip of the disinfectant administration device (140) such that the disinfectant administration device (140) is able to introduce disinfectant into the disinfectant reservoir (130).

12. The PD system (10) of Claim 9, which includes a source of disinfectant and a tube leading from the source of disinfectant to the disinfectant reservoir (130).

13. The PD system (10) of Claims 1 or 12, wherein the external tubing surface cleaning unit (110) is located within the housing (22).

14. The PD system (10) of Claim 13, wherein the housing (22) provides an opening (22o) allowing an openable sealed closure (136) of the external tubing surface cleaning unit (110) to be accessed for refilling disinfectant.

15. The PD system (10) of Claim 1, wherein the external tubing surface cleaning unit (110) is attachable to and detachable from the housing (22).

16. The PD system (10) of Claim 15, wherein the housing (22) defines at least one mating aperture, and wherein the external tubing surface cleaning unit (110) includes at least one feature sized and shaped to snap-fit within the at least one mating aperture.

17. The PD system (10) of Claim 1, wherein the external tubing surface cleaning unit (110) includes a disinfectant reservoir (130), and wherein the system (10) includes at least one sensor (88) positioned and arranged to detect a level of disinfectant within the disinfectant reservoir (130).

18. The PD system (10) of Claim 17, which includes a control unit (100) and a user interface (108), the at least one sensor outputting to the control unit (100), and wherein the control unit (100) is configured to cause the user interface (108) to prompt for a disinfectant refill when an output from the at least one sensor (88) indicates a low disinfectant level within the disinfectant reservoir (130).

19. The PD system (10) of Claim 1, which includes at least one ultraviolet ("UV") light (92) positioned and arranged to disinfect the exterior of the reusable PD fluid line (24a to 24d) as it is pulled into the housing (22).

20. The PD system (10) of Claim 1, which includes a source of disinfectant and a tube leading from the source of disinfectant into the external tubing surface cleaning unit (110) so as to be able to apply disinfectant to at least the portion of the exterior of the reusable PD fluid line (24a to 24d) as it is pulled into the housing (22).

21. A peritoneal dialysis ("PD") system (10) comprising:
a PD machine (20) including a housing (22);
a reusable PD fluid line (24a to 24d) positioned and arranged to be pulled into the housing (22); and
an external tubing surface cleaning unit (110) including a scrubber (120) sized and shaped to extend around and contact the reusable PD fluid line (24a to 24d) to remove debris from at least a portion of an exterior (28d) of the reusable PD fluid line (24a to 24d) as it is pulled into the housing (22).

22. A method for cleaning an exterior of a reusable PD fluid line (24a to 24d) comprising:
enabling a reusable PD fluid line (24a to 24d) to be retracted into a PD fluid machine (20); and
causing debris from at least a portion of an exterior (28d) of the reusable PD fluid line (24a to 24d) to be removed as the reusable PD fluid line (24a to 24d) is retracted into the PD fluid machine (20) using a scrubber (120) sized and shaped to extend around and contact the reusable PD fluid line (24a to 24d).

## Patentansprüche

1. Peritonealdialysesystem ("PD") (10), umfassend:
ein Gehäuse (22);
eine in dem Gehäuse (22) beherbergte PD-Fluidpumpe (70);
eine wiederverwendbare PD-Fluidleitung (24a bis 24d), die mit der PD-Fluidpumpe (70) strömungsverbunden ist, wobei die wiederverwendbare PD-Fluidleitung (24a bis 24d) so positioniert und angeordnet ist, dass sie in das Gehäuse (22) eingezogen werden kann; und
eine externe Schlauchoberflächen-Reinigungseinheit (110) einschließlich eines Kontaktreinigungselements (120), das so dimensioniert und geformt ist, dass es sich um die wiederverwendbare PD-Fluidleitung (24a bis 24d) erstreckt und mit dieser in Kontakt steht, um Verunreinigungen von mindestens einem Abschnitt einer Außenfläche (28d) der wiederverwendbaren PD-Fluidleitung (24a bis 24d) zu entfernen, während diese in das Gehäuse (22) eingezogen wird.

2. PD-System (10) nach Anspruch 1, wobei die wiederverwendbare PD-Fluidleitung (24a bis 24d) eine wiederverwendbare Patientenleitung (28) ist.

3. PD-System (10) nach Anspruch 2, wobei die wiederverwendbare Patientenleitung (28) eine wiederverwendbare Doppellumen-Patientenleitung (28) ist.

4. PD-System (10) nach Anspruch 1, das eine im Gehäuse (22) angeordnete Schlauchtrommel (80) einschließt, wobei die Schlauchtrommel (80) konfiguriert ist, um die wiederverwendbare PD-Fluidleitung (24a bis 24d) in das Gehäuse (22) einzuziehen.

5. PD-System (10) nach Anspruch 1, das eine Steuereinheit (100) umfasst, die konfiguriert ist, um die Durchführung einer Desinfektionssequenz zu bewirken, in der ein Innenraum der wiederverwendbaren PD-Fluidleitung (24a bis 24d) desinfiziert wird, und wobei die wiederverwendbare PD-Fluidleitung (24a bis 24d) in das Gehäuse (22) eingezogen wird, bevor die Desinfektionssequenz durchgeführt wird.

6. PD-System (10) nach Anspruch 1, wobei das Kontaktreinigungselement (120) ein schaumstoff- oder faserbasiertes Kontaktreinigungselement ist, das ausgebildet ist, um Desinfektionsmittel auf mindestens den Abschnitt der Außenseite (28d) der wiederverwendbaren PD-Fluidleitung (24a bis 24d) aufzubringen, während sie in das Gehäuse (22) eingezogen wird.

7. PD-System (10) nach Anspruch 6, wobei das schaumstoff- oder faserbasierte Kontaktreinigungselement (120) einen zylindrischen Abschnitt (122) einschließt, der so bemessen und geformt ist, dass es sich um die wiederverwendbare PD-Fluidleitung (24a bis 24d) erstreckt und Desinfektionsmittel auf diese aufbringt, und eine Desinfektionsmittelapplikationsverlängerung (124), die sich von dem zylindrischen Abschnitt (122) in ein Desinfektionsmittelreservoir (130) erstreckt, wobei die Desinfektionsmittelapplikationsverlängerung (124) ermöglicht, dass Desinfektionsmittel aus dem Desinfektionsmittelreservoir (130) zu dem zylindrischen Abschnitt (122) fließt.

8. PD-System (10) nach Anspruch 7, wobei der zylindrische Abschnitt (122) des schaumstoff- oder faserbasierten Kontaktreinigungselements (120) von einem Kragen (112) gestützt wird, wobei sich das Desinfektionsmittelreservoir (130) von dem Kragen (112) aus erstreckt.

9. PD-System (10) nach Anspruch 6, wobei die externe Schlauchoberflächen-Reinigungseinheit (110) ein Desinfektionsmittelreservoir (130) einschließt, das konfiguriert ist, um ein Desinfektionsmittel zur Aufbringung auf mindestens den Abschnitt der Außenseite (28d) der wiederverwendbaren PD-Fluidleitung (24a bis 24d) aufzunehmen, während diese in das Gehäuse (22) eingezogen wird.

10. PD-System (10) nach Anspruch 9, wobei das Desinfektionsmittelreservoir (130) einen öffenbaren dichtenden Verschluss (136) einschließt, der konfiguriert ist, um zu ermöglichen, dass Desinfektionsmittel in das Desinfektionsmittelreservoir (130) eingebracht wird.

11. PD-System (10) nach Anspruch 9, das eine Desinfektionsmittelverabreichungsvorrichtung (140) einschließt, wobei der öffenbare dichtende Verschluss (136) konfiguriert ist, um eine Spitze der Desinfektionsmittelverabreichungsvorrichtung (140) aufzunehmen, sodass die Desinfektionsmittelverabreichungsvorrichtung (140) in der Lage ist, Desinfektionsmittel in das Desinfektionsmittelreservoir (130) einzubringen.

12. PD-System (10) nach Anspruch 9, das eine Desinfektionsmittelquelle und einen Schlauch einschließt, der von der Desinfektionsmittelquelle zu dem Desinfektionsmittelreservoir (130) führt.

13. PD-System (10) nach Anspruch 1 oder 12, wobei die externe Schlauchoberflächen-Reinigungseinheit (110) innerhalb des Gehäuses (22) angeordnet ist.

14. PD-System (10) nach Anspruch 13, wobei das Gehäuse (22) eine Öffnung (22o) bereitstellt, die den Zugriff auf einen öffenbaren dichtenden Verschluss (136) der externen Schlauchoberflächen-Reinigungseinheit (110) zum Nachfüllen von Desinfektionsmittel ermöglicht.

15. PD-System (10) nach Anspruch 1, wobei die externe Schlauchoberflächen-Reinigungseinheit (110) an dem Gehäuse (22) anbringbar und von diesem abnehmbar ist.

16. PD-System (10) nach Anspruch 15, wobei das Gehäuse (22) mindestens eine Passöffnung definiert, und wobei die externe Schlauchoberflächen-Reinigungseinheit (110) mindestens ein Merkmal einschließt, das so bemessen und geformt ist, dass es in die mindestens eine Passöffnung einrastet.

17. PD-System (10) nach Anspruch 1, wobei die externe Schlauchoberflächen-Reinigungseinheit (110) ein Desinfektionsmittelreservoir (130) einschließt, und wobei das System (10) mindestens einen Sensor (88) einschließt, der positioniert und angeordnet ist, um einen Füllstand an Desinfektionsmittel innerhalb des Desinfektionsmittelreservoirs (130) zu erfassen.

18. PD-System (10) nach Anspruch 17, das eine Steuereinheit (100) und eine Bedienerschnittstelle (108) einschließt, wobei der mindestens eine Sensor ein Ausgangssignal an die Steuereinheit (100) liefert, und wobei die Steuereinheit (100) konfiguriert ist, um zu bewirken, dass die Bedienerschnittstelle (108) zu einem Nachfüllen von Desinfektionsmittel auffordert, wenn ein Ausgangssignal von dem mindestens einen Sensor (88) einen niedrigen Desinfektionsmittelstand innerhalb des Desinfektionsmittelreservoirs (130) anzeigt.

19. PD-System (10) nach Anspruch 1, das mindestens ein ultraviolettes Licht ("UV"-Licht) (92) einschließt, das positioniert und angeordnet ist, um die Außenseite der wiederverwendbaren PD-Fluidleitung (24a bis 24d) zu desinfizieren, während sie in das Gehäuse (22) eingezogen wird.

20. PD-System (10) nach Anspruch 1, das eine Quelle für Desinfektionsmittel und einen Schlauch einschließt, der von der Quelle für Desinfektionsmittel in die Schlauchoberflächen-Reinigungseinheit (110) führt, um Desinfektionsmittel auf mindestens den Abschnitt der Außenseite der wiederverwendbaren PD-Fluidleitung (24a bis 24d) aufbringen zu können, während diese in das Gehäuse (22) eingezogen wird.

21. Peritonealdialysesystem ("PD") (10), umfassend:
eine PD-Maschine (20) einschließlich eines Gehäuses (22);
eine wiederverwendbare PD-Fluidleitung (24a bis 24d), die positioniert und angeordnet ist, um in das Gehäuse (22) eingezogen zu werden; und
eine externe Schlauchoberflächen-Reinigungseinheit (110) einschließlich eines Kontaktreinigungselements (120), das so dimensioniert und geformt ist, dass es sich um die wiederverwendbare PD-Fluidleitung (24a bis 24d) erstreckt und mit dieser in Kontakt steht, um Verunreinigungen von mindestens einem Abschnitt einer Außenfläche (28d) der wiederverwendbaren PD-Fluidleitung (24a bis 24d) zu entfernen, während diese in das Gehäuse (22) eingezogen wird.

22. Verfahren zum Reinigen einer Außenseite einer wiederverwendbaren PD-Fluidleitung (24a bis 24d), umfassend:
Ermöglichen des Zurückziehens einer wiederverwendbaren PD-Fluidleitung (24a bis 24d) in eine PD-Fluidmaschine (20); und
Bewirken, dass Verunreinigungen von mindestens einem Abschnitt einer Außenseite (28d) der wiederverwendbaren PD-Fluidleitung (24a bis 24d) entfernt werden, während die wiederverwendbare PD-Fluidleitung (24a bis 24d) in die PD-Fluidmaschine (20) zurückgezogen wird, unter Verwendung eines Kontaktreinigungselements (120), das so bemessen und geformt ist, dass es sich um die wiederverwendbare PD-Fluidleitung (24a bis 24d) erstreckt und mit dieser in Kontakt steht.

## Revendications

1. Système de dialyse péritonéale (« DP ») (10) comprenant :
un logement (22) ;
une pompe à fluide DP (70) logée par le logement (22) ;
une ligne de fluide DP réutilisable (24a à 24d) en communication fluidique avec la pompe à fluide DP (70), la ligne de fluide DP réutilisable (24a à 24d) étant positionnée et agencée pour être tirée dans le logement (22) ; et
une unité de nettoyage de surface de tubulure externe (110) comportant un système de récurage (120) dimensionné et mis en forme pour s'étendre autour de, et venir en contact avec, la ligne de fluide DP réutilisable (24a à 24d) pour éliminer des débris d'au moins une partie d'un extérieur (28d) de la ligne de fluide DP réutilisable (24a à 24d) lorsqu'elle est tirée dans le logement (22).

2. Système DP (10) selon la revendication 1, dans lequel la ligne de fluide DP réutilisable (24a à 24d) est une ligne de patient réutilisable (28).

3. Système DP (10) selon la revendication 2, dans lequel la ligne de patient réutilisable (28) est une ligne de patient réutilisable (28) à double lumière.

4. Système DP (10) selon la revendication 1, qui comporte un dévidoir (80) localisé au sein du logement (22), le dévidoir (80) étant conçu pour tirer la ligne de fluide DP réutilisable (24a à 24d) dans le logement (22).

5. Système DP (10) selon la revendication 1, qui comporte une unité de commande (100) configurée pour amener une séquence de désinfection à être mise en œuvre dans laquelle un intérieur de la ligne de fluide DP réutilisable (24a à 24d) est désinfecté, et dans lequel la ligne de fluide DP réutilisable (24a à 24d) est tirée dans le logement (22) avant que la séquence de désinfection ne soit mise en œuvre.

6. Système DP (10) selon la revendication 1, dans lequel le système de récurage (120) est un système de récurage en mousse ou fibreux formé de façon à appliquer du désinfectant au moins à la partie de l'extérieur (28d) de la ligne de fluide DP réutilisable (24a à 24d) à mesure qu'elle est tirée dans le logement (22).

7. Système DP (10) selon la revendication 6, dans lequel le système de récurage (120) en mousse ou fibreux comporte une partie cylindrique (122) dimensionnée et mise en forme pour s'étendre autour et appliquer du désinfectant à la ligne de fluide DP réutilisable (24a à 24d), et une extension d'application de désinfectant (124) s'étendant à partir de la partie cylindrique (122) dans un réservoir de désinfectant (130), dans lequel l'extension d'application de désinfectant (124) permet à du désinfectant provenant du réservoir de désinfectant (130) de s'écouler vers la partie cylindrique (122).

8. Système DP (10) selon la revendication 7, dans lequel la partie cylindrique (122) du système de récurage (120) en mousse ou fibreux est supportée par un collier (112), le réservoir de désinfectant (130) s'étendant à partir du collier (112).

9. Système DP (10) selon la revendication 6, dans lequel l'unité de nettoyage de surface de tubulure externe (110) comporte un réservoir de désinfectant (130) conçu pour contenir un désinfectant pour application à au moins la partie de l'extérieur (28d) de la ligne de fluide DP réutilisable (24a à 24d) à mesure qu'elle est tirée dans le logement (22).

10. Système DP (10) selon la revendication 9, dans lequel le réservoir de désinfectant (130) comporte une fermeture scellée ouvrable (136) conçue pour permettre à du désinfectant d'être introduit dans le réservoir de désinfectant (130).

11. Système DP (10) selon la revendication 9, qui comporte un dispositif d'administration de désinfectant (140), dans lequel la fermeture scellée ouvrable (136) est conçue pour recevoir un bout du dispositif d'administration de désinfectant (140) de telle sorte que le dispositif d'administration de désinfectant (140) est capable d'introduire du désinfectant dans le réservoir de désinfectant (130).

12. Système DP (10) selon la revendication 9, qui comporte une source de désinfectant et un tube menant de la source de désinfectant au réservoir de désinfectant (130).

13. Système DP (10) selon les revendications 1 ou 12, dans lequel l'unité de nettoyage de surface de tubulure externe (110) est localisée au sein du logement (22).

14. Système DP (10) selon la revendication 13, dans lequel le logement (22) fournit une ouverture (22o) permettant d'accéder à une fermeture scellée ouvrable (136) de l'unité de nettoyage de surface de tubulure externe (110) pour le réapprovisionnement de désinfectant.

15. Système DP (10) selon la revendication 1, dans lequel l'unité de nettoyage de surface de tubulure externe (110) peut être attachée au, et détachée du, logement (22).

16. Système DP (10) selon la revendication 15, dans lequel le logement (22) définit au moins un orifice d'accouplement, et dans lequel l'unité de nettoyage de surface de tubulure externe (110) comporte au moins une caractéristique dimensionnée et mise en forme pour s'encliqueter au sein de l'au moins un orifice d'accouplement.

17. Système DP (10) selon la revendication 1, dans lequel l'unité de nettoyage de surface de tubulure externe (110) comporte un réservoir de désinfectant (130), et dans lequel le système (10) comporte au moins un capteur (88) positionné et agencé pour détecter un niveau de désinfectant au sein du réservoir de désinfectant (130).

18. Système DP (10) selon la revendication 17, qui comporte une unité de commande (100) et une interface utilisateur (108), l'au moins un capteur délivrant en sortie vers l'unité de commande (100), et dans lequel l'unité de commande (100) est configurée pour amener l'interface utilisateur (108) à demander un réapprovisionnement de désinfectant lorsqu'une sortie provenant de l'au moins un capteur (88) indique un faible niveau de désinfectant au sein du réservoir de désinfectant (130).

19. Système **DP** (10) selon la revendication 1, qui comporte au moins une lumière ultraviolette (« UV ») (92) positionnée et agencée pour désinfecter l'extérieur de la ligne de fluide **DP** réutilisable (24a à 24d) à mesure qu'elle est tirée dans le logement (22).

20. Système **DP** (10) selon la revendication 1, qui comporte une source de désinfectant et un tube menant de la source de désinfectant vers l'unité de nettoyage de surface de tubulure externe (110) de façon à pouvoir appliquer du désinfectant au moins à la partie de l'extérieur de la ligne de fluide **DP** réutilisable (24a à 24d) à mesure qu'elle est tirée dans le logement (22).

21. Système de dialyse péritonéale (« **DP** ») (10) comprenant :
une machine **DP** (20) comportant un logement (22) ;
une ligne de fluide **DP** réutilisable (24a à 24d) positionnée et agencée pour être tirée dans le logement (22) ; et
une unité de nettoyage de surface de tubulure externe (110) comportant un système de récurage (120) dimensionné et mis en forme pour s'étendre autour de, et venir en contact avec, la ligne de fluide **DP** réutilisable (24a à 24d) pour éliminer des débris d'au moins une partie d'un extérieur (28d) de la ligne de fluide **DP** réutilisable (24a à 24d) lorsqu'elle est tirée dans le logement (22).

22. Procédé permettant de nettoyer un extérieur d'une ligne de fluide **DP** réutilisable (24a à 24d) comprenant :
le fait de permettre à une ligne de fluide **DP** réutilisable (24a à 24d) d'être rétractée dans une machine à fluide **DP** (20) ; et
le fait d'amener des débris provenant d'au moins une partie d'un extérieur (28d) de la ligne de fluide **DP** réutilisable (24a à 24d) à être éliminés à mesure que la ligne de fluide **DP** réutilisable (24a à 24d) est rétractée dans la machine à fluide DP (20) à l'aide d'un système de récurage (120) dimensionné et mis en forme pour s'étendre autour de, et venir en contact avec, la ligne de fluide DP réutilisable (24a à 24d).
